# EUROPÄISCHE PATENTSCHRIFT

(11) **EP 2 994 200 B1**
(45) Veröffentlichungstag und Bekanntmachung des Hinweises auf die Patenterteilung: **11.05.2022**
(21) Anmeldenummer: 14711991.1
(22) Anmeldetag: 25.03.2014
(51) Int. Cl.: A61Q 5/06, A61K 8/81, A61K 8/04, A61K 8/33, A61K 8/39, A61K 8/41, A61Q 19/00

(54) **KOSMETISCHES ODER DERMATOLOGISCHES AEROSOL GEL**
COSMETIC OR DERMATOLOGICAL AEROSOL GEL
AEROSOL GEL COSMETIC OU DERMATIOLOGIC

(30) Priorität: 08.05.2013 DE 102013208483
(43) Veröffentlichungstag der Anmeldung: 16.03.2016
(73) Patentinhaber: Beiersdorf AG, 20253 Hamburg (DE)
(72) Erfinder: SPAN, Philipp, 22529 Hamburg (DE); KRIEGER, Wiebke, 20259 Hamburg (DE); FERNKVIST, Anna, S-74145 Knivsta (SE)
(74) Vertreter: Beiersdorf AG
(86) Internationale Anmeldenummer: PCT/EP2014/055918
(87) Internationale Veröffentlichungsnummer: WO 2014/180598

(56) Entgegenhaltungen:
- EP-A1- 1 192 931
- EP-A2- 2 098 216
- WO-A1-2008/031892
- DE-A1- 10 029 262
- DE-A1-102010 061 899
- DE-U1-202010 015 742
- US-A1- 2004 141 926
- Anonymous: "GNPD - Crackling Styling Gel", , 1. April 2011 (2011-04-01), XP055129986, Gefunden im Internet: URL:http://www.gnpd.com/sinatra/recordpage /1520104/from_search/uIznrIxYXr/ [gefunden am 2014-07-18]

## Beschreibung

Die Erfindung ist ein kosmetisches oder dermatologisches Aerosol Gel. Das Gel verursacht keine Fadenbildung, keine Röllchenbildung und kein klebriges Gefühl auf der Haut.

Gelartige Formulierungen sind häufig im Markt zu finden. Für den Verbraucher stellen u. a. die gute Handhabbarkeit und Sensorik wichtige Eigenschaften gelartiger Produkte dar. Bei der Zusammenstellung der Formulierung muss deshalb darauf geachtet werden, dass die Viskosität der Formulierung angemessen ist. Sowohl zu dicke, als auch zu dünne Formeln lassen sich schwierig in den Händen und auf der Haut verteilen.

Zudem ist die Sprühapplikation eines hochviskosen Gels mit Problemen verbunden.

Die hohe Viskosität schränkt die Auswahl der zur Verfügung stehenden Sprühköpfe ein und kann zu Verstopfungen des Sprühkopfes führen. Das verwendete Ventil muss ebenfalls passend zu dem Applikationssystem gewählt werden und kann einen signifikanten Einfluss auf die Produktapplikation haben. Unter Umständen kann es durch eine fehlerhafte Viskositätseinstellung in Kombination mit den Packmitteln auch zu einer als mangelhaft zu beurteilenden Restentleerung der Dose kommen.

Sowohl für die Rheologie der Formulierung als auch für Sensorik und Haptik ist die Auswahl des Verdickersystems von enormer Bedeutung.

Gelförmige Zubereitungen des Standes der Technik weisen häufig eine Fadenbildung oder Röllchenbildung auf der Haut auf und lassen mitunter ein klebriges Gefühl auf der Haut entstehen. Das Phänomen der Röllchenbildung wird verursacht durch Verdickerrückstände auf der Haut bzw. durch die Auswahl einer ungeeigneten Kettenlänge des Verdickers. Dies wird vom Verbraucher negativ bewertet.

DE 10 2007 029 734 A1 beschreibt transparente oder translucente kosmetische Zubereitungen enthaltend u.a. ein vernetztes Polymer aus Vinylpyrrolidon und Acrylsäure.

DE 10 2006 032 015 A1 und DE 10 2009 034 115 A1 stellen Stand der Technik Dokumente dar in denen Verdicker u.a. auf Polyacrylbasis und Treibmittel wie u.a. Dimethylether genannt werden. Es sind jedoch keine spezifischen Kombinationen offenbart und es können beliebige Kombinationen von Treibgas/Treibgasen verwendet werden. Der Fachmann kann anhand dieser Dokumente das Treibgas scheinbar frei wählen.

Aerosolzubereitungen weisen häufig, gewünscht oder unerwünscht, einen Knister- oder Crackling-Effekt auf. Dieser lässt sich beschreiben als haptischen Effekt, der sich dadurch ergibt, dass sich beim Auftragen und Verteilen der zumeist gelartigen Formulierung in den Händen oder auf der Haut durch das Verdampfen des Treibmittels aufgrund der Körperwärme eine große Vielzahl von kleinen Gasbläschen bildet. Diese Gasbläschen vergrößern sich relativ schnell und zerplatzen unter Erzeugung eines knisternden Geräusches und eines angenehm prickelnden Gefühls der zerplatzenden Bläschen auf der Haut.

DE 20 2010 015 742 U1 beschreibt ein haarfixierendes Knistergel umfassend ein oder mehrere Treibgase, wenigstens einen Alkohol, verdickende Polymere, wenigstens ein haarfixierendes Polymer und gegebenenfalls weitere Nebenkomponenten.

Als Treibgas wird bevorzugt Dimethylether verwendet. Abmischungen von Dimethylether mit anderen Gasen sind ebenfalls dargestellt. Das Treibgas ist in der Wirkstofflösung gelöst oder lässt sich durch leichtes Schütteln mit der Formulierung vermischen.

DE 100 29 262 A1 beschreibt eine Aerosol-Körperlotion mit Knistereffekt auf der Grundlage einer kosmetische Wirkstoffe enthaltenden wässrig-alkoholischen Trägerflüssigkeit, die mindestens einen Emulgator, mindestens einen Emulsions-Stabilisator, mindestens ein Verdickungsmittel und ein Aerosol-Treibmittel oder Aerosol-Treibmittelgemisch mit einem Siedepunkt von -15°C bis +40°C enthält, sowie ein Verfahren zu ihrer Herstellung.

In der WO 2008031892 A1 werden Zubereitungen für die Haarkosmetik, insbesondere für die Herstellung von Aerosolschäumen, beschrieben, die eine Vielzahl an Verdickern auf Polyacrylbasis umfassen und als deren Treibmittel Dimethylether gewählt werden kann.

Wünschenswert ist es eine gelartige Zubereitung bereit zu stellen, die die Nachteile der Zubereitungen des Standes der Technik nicht aufweist.

Insbesondere ist es wünschenswert Aerosol Gele zur Verfügung zu stellen, die keine Fadenbildung, keine Röllchenbildung und kein klebriges Gefühl auf der Haut verursachen.

Die Erfindung ist ein kosmetisches oder dermatologisches Aerosol Gel umfassend Wasser, 0,05 bis 5 Gew.% eines Verdickers und 5 bis 50 Gew.% Dimethylether als Treibmittel entsprechend Anspruch 1. Der Verdickeranteil bezieht sich dabei auf die Gesamtmasse des Gels ohne Treibmittel, der Anteil des Treibmittels auf die Gesamtmasse inklusive Treibmittel.

Das Gel umfasst keine weiteren Treibmittel und/oder keine weiteren Verdicker. Vorteilhaft sind lediglich nur Dimethylether (DME) und nur ein Verdicker in der Gelzubereitung enthalten.

Treibmittel, auch Treibgase genannt, sind Hilfsmittel zur Zerstäubung von Teilchen. Man versteht im Allgemein verflüssigte oder verdichtete Gase oder Stoffe darunter. Zu den bekanntesten Treibgasen zählen:
- FCKW (z. B. Trichlorfluormethan, Dichlordifluormethan)
- Kohlenwasserstoffe (z. B. Propan, Butan)
- Dimethylether
- Kohlendioxid
- Stickstoff
- Luft

Das früher in Sprühdosen häufig verwendete FCKW ist mittlerweile verboten, da es als höchst umweltschädlich gilt.

Als Verdickungsmittel werden Stoffe verstanden, die alleinig die Viskosität einer Zubereitung bei gleicher Temperatur erhöhen.

Ein Stoff, der grundsätzlich nicht verdickend wirkt und wirken kann, ist patentgemäß und für den Fachmann nicht unter dem Begriff Verdickungsmittel zu verstehen. Verdickung bedeutet dabei, dass durch Zugabe eines Verdickungsmittels zu einer Zubereitung deren Viskosität erhöht wird. Findet allein durch die Zugabe dieses Mittels keinerlei Viskositätserhöhung statt ist dieses Mittel als nicht verdickend anzusehen. Zu den bekannten Verdickungsmitteln zählen Pektine, Guar, Johannisbrotkernmehl, Carrageen, Celluloseether, Polyvinylalkohol, Silikate, Polyacrylate und Polyacrylamide.

Ein Gel wird in der Regel als ein feindisperses System aus mindestens einer festen und einer flüssigen Phase definiert. Die feste Phase bildet dabei ein schwammartiges, dreidimensionales Netzwerk, dessen Poren durch eine Flüssigkeit (*Lyogel*) oder ein Gas (*Xerogel*) ausgefüllt sind. Ist das Netzwerk porös und Luft oder Treibmittel das eingelagerte Gas, so wird das Gel auch als *Aerogel* oder Aerosolgel bezeichnet.

Die Zubereitung umfasst im Wesentlichen nur ein Treibmittel, Dimethylether. Das Treibmittel ist in der Zusammensetzung des erfindungsgemäßen Produktes in einer Menge von 5 bis 50 Gew.%, besonders bevorzugt von 10 bis 35 Gew.%, bezogen auf die Gesamtzusammensetzung incl. des Treibmittels, enthalten. Sind lediglich geringe Mengen (<5%) anderer Treibmittel, in Spuren oder eingeschleppt, in der Aerosolgelzubereitung enthalten, so gilt die Gelzubereitung weiterhin als nur mit einem einzigen Treibmittel versehen.

Gegenüber bekannten Formulierungen ist hierbei eine spezielle Kombination von einer Gel-Formulierung und einem spezifischen Treibgas gefunden worden, die den Nachteilen des Standes Technik abhilft.

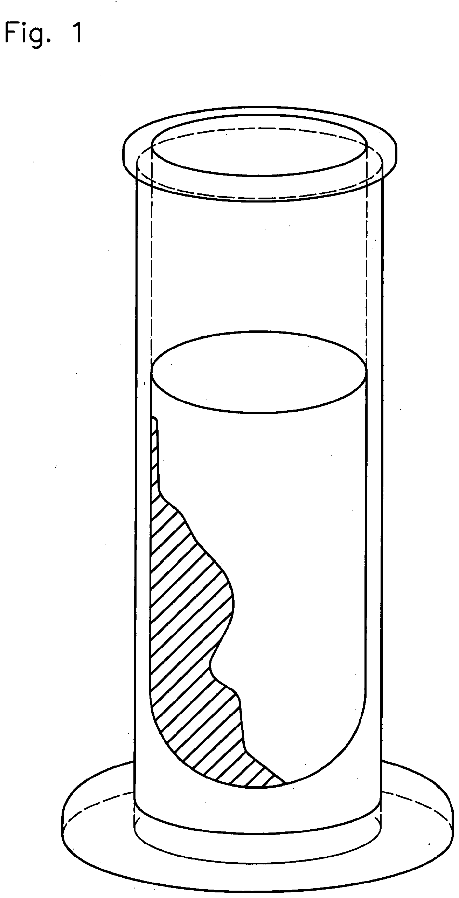

Vergleichsuntersuchungen zeigen, dass mit üblichen Treibmitteln, wie einem Gemisch aus Propan-Butan, die erfindungsgemäßen Vorteile nicht erzielt werden können. Explizit lassen sich die erfindungsgemäßen Formulierungen, wie in den Beispielen aufgeführt, mit Propan/Butan Treibmittelgemische nicht in Lösung bringen. Dazu wurde die im Beispiel 1 aufgeführte Formel mit 30% Gas, bestehend aus einem Gemisch aus Propan und Butan (2.7 bar) begast. Eine Vermischung des Gases mit der Formel konnte, wie in der Abbildung 1 ersichtlich nicht, nicht beobachtet werden. Auch durch mehrmaliges Schütteln konnte eine Homogenisierung nicht erreicht werden.

Abbildung 1 und 1a zeigt ein Glaszylinderaerosol mit dem Gel und 30% Propan/Butan Gemisch (2.7 bar). Auch nach mehrmaligem Schütteln erfolgte keine Vermischung, das Gel klebte an der Glaswandung. Abbildung 1a zeigt die Gelreste schwarz an der Wandung.

Eine Sprühapplikation wäre damit ausgeschlossen.

Als Verdicker werden Verbindungen auf Basis von Polyacrylsäure, wie Carbomere, Carbopole, vernetzte Polymere aus Vinylpyrrolidon und Acrylsäure etc., bevorzugt verwendet.

Durch den Einsatz eines Verdickers ist es möglich auch nach der Zugabe von Treibgas eine stabile Formel beizubehalten. Eine Zerstörung der Gel-Formel durch Ausfallen des Verdickers zeigt sich nicht.

Erfindungsgemäß ist vorteilhaft nur ein einziger Verdicker in der Zubereitung enthalten.

Überraschenderweise zeigte sich mit nur einem Treibmittel Dimethylether und nur einem Verdicker, Acrylic Acid/VP Crosspolymer, dass die erhaltene erfindungsgemäßen Aerosolgele alle benötigten Anforderungen, wie kein Röllchenbildung, der Viskositätsbereich ist einstellbar, der pH-Wert ist stabil, das Gel ist geruchsneutral, erfüllt werden. Vor allem aber zeigt das Gel keine bzw. nur eine geringe Klebrigkeit auf Haut.

Erfindungsgemäß ist als Verdicker Acrylic Acid/VP Crosspolymer zu wählen. Acrylic Acid/VP Crosspolymer (CAS No: 527685-31-0) ist als UltraThix P-100 der Firma ISP bekannt.

Die Verwendung von Acrylic Acid/VP Crosspolymer in Aerosolgelzubereitungen umfassend als Treibmittel nur Dimethylether führt zur Verringerung der Klebrigkeit der Zubereitung auf der Haut.

Probleme, wie Ausfällung des Verdickers in Kombination mit einem Treibmittel und somit kompletter Viskositätsverlust sowie ein starker Viskositätsabbau bei hohen Mengen an Alkohol, zeigen sich erfindungsgemäß nicht.

Es werden keine Stylingpolymere aus Haaranwendungszubereitungen, die sich störend auf die Klebrigkeit des Gels auswirken können, in die erfindungsgemäße Gelzubereitung eingearbeitet.

Der Verdicker wird dem erfindungsgemäßen Gel im Bereich von 0,05 bis 5 Gew.%, insbesondere im Bereich von 0,1 bis 2 Gew.%, bezogen auf die Gesamtmasse des Gels ohne Treibmittel zugesetzt.

Der Wasseranteil des erfindungsgemäßen Gels liegt vorteilhaft im Bereich von etwa 20 bis 70 Gew.%, insbesondere 20 bis 70 Gew.%, vorteilhaft im Bereich von 40 bis 55 Gew.%, bezogen auf die Gesamtmasse des Gels ohne Treibmittel.

Das Gel umfasst vorteilhaft Alkohole. Als Alkohol können bei Raumtemperatur flüssige Alkohole eingesetzt werden, wie sie als Lösemittel, insbesondere Ethanol, bekannt sind.

Der Anteil an einem oder mehreren Alkoholen liegt vorteilhaft im Bereich von etwa bis zu 50 Gew.%, insbesondere bei 20 bis 50 Gew.%, hauptsächlich im Bereich von 30 bis 45 Gew.%, bezogen auf die Gesamtmasse der Gels ohne Treibmittel. Bevorzugt umfasst die Gelzubereitung Ethanol, insbesondere bis zu einem Anteil von 50 Gew.%.

In einer anderen Ausführungsform wird auf den Zusatz von Ethanol verzichtet.

Der Einsatz eines Emulgators ist zur Herstellung der Gel-Formulierung nicht zwingend notwendig. Es kann daher vorteilhaft auf den Einsatz von Emulgatoren verzichtet werden. Dennoch ist es möglich einen oder mehrere Emulgatoren und deren Einsatzkonzentration in Abhängigkeit von den ein zuformulierenden Ölen/Silikonen/Extrakten etc. und deren Einsatzkonzentrationen einzusetzen. Bevorzugt kann als Emulgator Polyglyceryl-10 Laurat eingesetzt werden.

Vorteilhaft sind ein oder mehrere Stabilisatoren im Gel vorhanden. Bevorzugt wird Aminomethylpropanol eingesetzt. Aminomethylpropanol wirkt puffernd, d.h. es stabilisiert den pH-Wert von kosmetischen Mitteln.

Die erfindungsgemäßen Gele weisen vorteilhaft eine Viskosität von 20 bis 400 dPas auf. Unterhalb von 20 dPas besteht die Gefahr, dass das Produkt beim Verreiben in den Händen zu Boden tropft und somit keine gute Handhabbarkeit mehr gegeben wäre. Die Obergrenze von 400 dPas kann, abhängig von weiteren rheologischen Eigenschaften des Gels, überschritten werden, wobei immer noch eine gute Verteilbarkeit in der Hand und im Haar vorhanden sein sollte.

Bevorzugt weisen die Gele eine Viskosität von 70-90 dPas auf. Die erfindungsgemäße Viskosität kann mit Hilfe eines Viscotester VT-02, der Firma Haake bei 25°C mit Hilfe des Drehkörpers 1 bzw. 2 und Ablesung der Skala 1 bzw. 2 bestimmt werden (entspricht einer Scherrate von 10 Pa/s).

Pflegenden Substanzen, z.B. Öle, Wachse, Silikone oder Glycerin können bevorzugt zur Verbesserung der Haptik und Hautpflege in die erfindungsgemäßen Gele eingearbeitet werden.

Besonders bevorzugt ist die Einarbeitung eines oder mehrerer Substanzen, die für ihre langanhaltenden kühlenden Eigenschaften bekannt sind, z.B. Menthol und dessen Derivate, Campher, Minze, Eukalyptus etc.

Als besonders bevorzugtes ätherisches Öl hat sich das Menthol erwiesen. Menthol ist Bestandteil des bekannten japanischen Pfefferminzöls (CAS: 20747-49-3). Wichtigstes Isomer ist (-)-Menthol. Menthol erzeugt beim Einreiben auf die Haut infolge Oberflächenanästhesierung und Reizung der kälteempfindlichen Nerven bei Migräne oder dergleichen ein angenehmes Kältegefühl. Erwiesenermaßen zeigen die betroffenen Hautpartien aber normale bzw. erhöhte Temperatur.

Als weitere bevorzugte ätherische Öle können Oleum Eucalypti, Oleum Menthae piperitae, Oleum camphoratum, Oleum Rosmarini, Oleum Thymi, Oleum Pini sibricum und Oleum Pini silverstris sowie die Terpene 1,8-Cineol und Levomethanol sowie Oleum Abietis albae, Oleum Anisi, Oleum Aurantii Floris, Oleum Bergamottae, Oleum Calendulae infusum, Oleum Caryophylli, Oleum Chamomillae, Oleum Cinnamomi ceylanici, Oleum Citri, Oleum Citronellae, Oleum Cupressi, Oleum Cymbopogonis, Oleum Jecoris, Oleum Lavendulae, Oleum Macidis, Oleum Majoranae, Oleum Melaleucae viridiflorae, Oleum Melissae, Oleum Menthae arvensis, Oleum Millefolium, Oleum Myrrhae, Oleum Myrte, Oleum Pini sibricum, Oleum Pinisilvestris, Oleum Salviae, Oleum Santali, Oleum Terebinthinae rectificat., Oleum Valerianae und Oleum Zingiberis eingesetzt werden.

Die ätherischen Öle werden einzeln oder in Kombination mit anderen zu einem Anteil von insgesamt 0,001 bis 10 Gew.%, insbesondere zu 0,01 bis 1 Gew.% in der Zubereitung eingesetzt, bezogen auf die Gesamtmasse der Zubereitung ohne Treibmittel.

Vorteilhaft können anstelle der scheinbar kühlend wirkend Substanzen auch Substanzen eingesetzt werden, die für wärmende Eigenschaften bekannt sind, wie beispielsweise HotAct (Vanillyl Buthyl Ether) und/oder Capsicum.

Die Zusammensetzung des erfindungsgemäßen Produktes kann darüber hinaus die für kosmetische Zubereitungen üblichen Zusatzbestandteile enthalten, z.B. Parfüm- und Duftstoffe, bevorzugt in einer Menge von 0,01 bis 1,5 Gew.%; Konservierungsstoffe, bevorzugt in einer Menge von 0,01 bis 5,0 Gew.%; Puffersubstanzen, wie z.B. Natriumcitrat oder Natriumphosphat in einer Menge von 0,1 bis 10,0 Gew.%; Pflegestoffe, wie z.B. Pflanzen- und Kräuterextrakte, Protein- und Seidenhydrolysate, Q10, Glycerylglucose, Antioxydantien, Lanolinderivate, bevorzugt in einer Menge von 0,1 bis 15 Gew.%; Lichtschutzmittel, Radikalfänger, Antischuppenwirkstoffe, bevorzugt in einer Menge von etwa 0,01 bis 12 Gew.%; Glanzgeber, Vitamine, Sensorikverbesserer und/oder rückfettende Stoffe. Diese Zusätze sind jedoch nur auszuwählen, sofern diese Zusätze keine Treibmittel sind und/oder Verdickungswirkungen zeigen.

Weitere Vorteile der erfindungsgemäßen Zubereitungen ergeben sich bei ihrer Herstellung.

Die Herstellung der erfindungsgemäßen Gele kann bei Raumtemperatur mit üblichen Laborgeräten stattfinden. Eine besondere Einhaltung der Zugabereihenfolge der verschiedenen Substanzen im Vergleich zu herkömmlichen Gelen ist nicht erforderlich. Ebenso vorteilhaft ist nach der Herstellung des Gels eine Begasung mit dem Treibmittel sofort möglich.

Nachfolgende Beispiele veranschaulichen die Erfindung. Die Zahlenangaben sind Gewichtsanteile bezogen auf die Gesamtmasse der Zubereitung ohne Treibmittel.

### Beispiel 1

| inci | Gew.% |
|---|---|
| Aminomethyl Propanol | 0,3 |
| Polyglyceryl-10 Laurate + Aqua | 0,4 |
| Alcohol Denat. | 35 |
| Aqua + Cl 42090 | 0,2 |
| Glycerin | 7 |
| Isopropyl Palmitate | 1 |
| Menthol | 0,4 |
| Parfum | 0,7 |
| Cyclomethicone | 2,3 |
| Vitis Vinifera Seed Oil | 0,5 |
| Acrylic Acid/VP Crosspolymer | 1,0 |
| Aqua | 51.2 |
| | 100 |

Anschließende Zugabe von 35 Gew.% DME, bezogen auf die Gesamtmasse incl. Treibmittel.

| inci | Gew.% |
|---|---|
| Aminomethyl Propanol | 0,1-1 |
| Polyglyceryl-10 Laurate + Aqua | 0,1-0,5 |
| Alcohol Denat. | 20-50 |
| Glycerin | 0,1-10 |
| Isopropyl Palmitate | 0,1-5 |
| Menthol | 0,1-1,5 |
| Parfum | 0,1-1,0 |
| Cyclomethicone | 0,1-3 |
| Vitis Vinifera Seed Oil | 0,1-3 |
| Acrylic Acid/VP Crosspolymer | 0,1-2 |
| Aqua | 20-70 |
| | 100 |

Anschließende Zugabe von 30 - 40 Gew.% DME, bezogen auf die Gesamtmasse incl. Treibmittel.

## Patentansprüche

1. Kosmetisches oder dermatologisches Aerosol Gel umfassend Wasser und 0,05 bis 5 Gew.% Acrylic Acid/VP Crosspolymer, bezogen auf die Gesamtmasse des Gels ohne Treibmittel, und 5 bis 50 Gew.% Dimethylether als Treibmittel, bezogen auf die Gesamtmasse des Aerosol Gels mit Treibmittel, **dadurch gekennzeichnet, dass** keine weiteren Treibmittel und/oder Verdicker enthalten sind und keine weiteren Stylingpolymere enthalten sind.

2. Gel nach Anspruch 1 **dadurch gekennzeichnet, dass** das Gel eine Viskosität von 20 bis 400 dPas aufweist, gemessen mit Viscotester VT-02, der Firma Haake bei 25°C mit Hilfe des Drehkörpers 1 bzw. 2 und Ablesung der Skala 1 bzw. 2.

3. Gel nach einem der vorstehenden Ansprüche umfassend bis zu 50 Gew.% Ethanol, bezogen auf die Gesamtmasse des Gels ohne Treibmittel.

4. Gel nach Anspruch 1 bis 2 **dadurch gekennzeichnet, dass** kein Ethanol enthalten ist.

5. Gel nach einem der vorstehenden Ansprüche umfassend ein oder mehrere Stabilisatoren, insbesondere Aminomethylpropanol.

6. Gel nach einem der vorstehenden Ansprüche umfassend ein oder mehrere ätherische Öle.

7. Gel nach einem der vorstehenden Ansprüche umfassend ein oder mehrere Emulgatoren.

8. Gel nach Anspruch 7 **dadurch gekennzeichnet, dass** als Emulgator Polyglyceryl-10 Laurat gewählt wird.

9. Verwendung von Acrylic Acid/VP Crosspolymer in Aerosolgelzubereitungen umfassend als Treibmittel nur Dimethylether zur Verringerung der Klebrigkeit der Zubereitung auf der Haut.

## Claims

1. Cosmetic or dermatological aerosol gel comprising water and 0.05 to 5% by weight acrylic acid/VP crosspolymer, based on the total mass of the gel without propellant, and 5 to 50% by weight dimethyl ether as propellant, based on the total mass of the aerosol gel including propellant, **characterized in that** no other propellants and/or thickeners are present and no other styling polymers are present.

2. Gel according to Claim 1, **characterized in that** the gel has a viscosity of 20 to 400 dPas, measured at 25°C with a VT-02 viscotester from Haake, using spindle 1 or 2 and readout from scale 1 or 2 respectively.

3. Gel according to either of the preceding claims comprising up to 50% by weight ethanol, based on the total mass of the gel without propellant.

4. Gel according to Claim 1 to 2, **characterized in that** no ethanol is present.

5. Gel according to any of the preceding claims comprising one or more stabilizers, particularly aminomethyl propanol.

6. Gel according to any of the preceding claims comprising one or more essential oils.

7. Gel according to any of the preceding claims comprising one or more emulsifiers.

8. Gel according to Claim 7, **characterized in that** the emulsifier selected is polyglyceryl-10 laurate.

9. Use of acrylic acid/VP crosspolymer in aerosol gel preparations comprising only dimethyl ether as propellant for reducing the stickiness of the preparation on the skin.

## Revendications

1. Gel aérosol cosmétique ou dermatologique comprenant de l'eau et 0,05 à 5 % en poids d'un polymère réticulé d'acide acrylique/VP, par rapport à la masse totale du gel sans propulseur, et 5 à 50 % en poids de diméthyléther en tant que propulseur, par rapport à la masse totale du gel aérosol avec le propulseur, **caractérisé en ce qu'**aucun autre propulseur et/ou épaississant n'est contenu et qu'aucun autre polymère de coiffage n'est contenu.

2. Gel selon la revendication 1, **caractérisé en ce que** le gel présente une viscosité de 20 à 400 dPas, mesurée avec un viscosimètre VT-02, de la société Haake à 25 °C à l'aide du corps rotatif 1 ou 2 et lecture de l'échelle 1 ou 2.

3. Gel selon l'une quelconque des revendications précédentes comprenant jusqu'à 50 % en poids d'éthanol, par rapport à la masse totale du gel sans propulseur.

4. Gel selon la revendication 1 à 2 **caractérisé en ce que** de l'éthanol n'est pas contenu.

5. Gel selon l'une quelconque des revendications précédentes comprenant un ou plusieurs stabilisants, en particulier de l'aminométhylpropanol.

6. Gel selon l'une quelconque des revendications précédentes comprenant une ou plusieurs huiles essentielles.

7. Gel selon l'une quelconque des revendications précédentes comprenant un ou plusieurs émulsifiants.

8. Gel selon la revendication 7 **caractérisé en ce que** du laurate de polyglycéryle-10 est choisi en tant qu'émulsifiant.

9. Utilisation d'un polymère réticulé d'acide acrylique/VP dans des préparations de gel aérosol comprenant en tant que propulseur seulement du diméthyléther pour la réduction du caractère collant de la préparation sur la peau.
